# EUROPEAN PATENT APPLICATION

(11) **EP 3 058 971 A1**
(43) Date of publication of application: **24.08.2016**
(21) Application number: 16156271.5
(22) Date of filing: 18.02.2016
(51) Int. Cl.: A61M 5/20, A61M 5/24, A61M 5/46

(54) **SYRINGES**

(30) Priority: 19.02.2015 GB 201502830; 31.03.2015 GB 201505507; 22.06.2015 GB 201510966; 28.10.2015 GB 201519092
(71) Applicant: Yutal Limited, Llandello Dyfed SA19 6BN (GB)
(72) Inventor: Lampard, Alice, Llandeilo, Dyfed SA19 6BN (GB); Livesey, Timothy Daniel, Packington, Leicestershire LE67 2TJ (GB)
(74) Representative: Swindell & Pearson Limited

(57) **Abstract**

A syringe (2) for injecting a substance to be administered into or through the skin of an animal or human comprising a reservoir (4) adapted to contain a substance to be administered to the animal or human, an introduction means (8) for introducing the substance to be administered into the animal or human, a dispensing means (12) suitable for dispensing a dose of the content of the reservoir from the reservoir into the animal or human via the introduction means, and a means (16) for actuating the dispensing means, characterised in that the syringe further comprises one or more of a means limiting the use of the syringe to one or other of the hands of the user, a means for monitoring the quantity of substance in the reservoir, a means to limit which substance may be introduced into the reservoir, the reservoir is reversibly removable from the syringe, or the syringe further comprises a means for marking the position where the introduction means has entered the skin.

## Description

This application relates to syringes, and in particular to syringes which can contain more than one dose of the substance to be injected in the reservoirs of the syringe.

Syringes are often multi use syringes where the syringe has a reservoir for holding the substance to be injected and that reservoir is sufficiently large that it may hold multiple doses of the substance. Such syringes may be used for the for testing of diseases, for example the testing of tuberculosis (TB) in cattle, goats, pigs, sheep, camelids and deer where the testing is performed by way of a skin test, or for inoculating multiple animals, for example a flock of sheep.

The syringes of the present invention will be mainly described in connection with the testing of TB in animals, but the functions and benefits of the syringes according to the present invention apply equally to other uses such as testing for other diseases, inoculation, or dosing of animals or humans.

Skin testing for TB in cattle, goats, pigs, sheep, camelids and deer is well known and is performed using a predetermined two stage methodology. This approach is intended to gain maximum consistency and accuracy in the results of the tests.

The first stage of the testing is performed in the following fashion:
The animal to be tested is selected and restrained, normally in a crush.

Two patches on the skin on the neck of the animal to be tested are shaved. The patches are located at a predetermined distance from each other in predetermined positions relative to each other and preferably in a predetermined position on the animal. For example, for cattle it is standard practice to locate one patch substantially above the other patch on the side of the neck when the animal is standing in the crush. For other animals the relative locations of the skin patches may be different but the essential methodology of the test remains the same. The thickness of the skin of the animal is measured in the middle of each shaved patch and recorded.

Into the middle of the predetermined shaved patch a dose of a first, normally avian, tuberculin is injected so that the tuberculin is delivered intradermally. Into the middle of the other predetermined shaved patch a dose of a second, normally bovine, tuberculin is injected so that the tuberculin is delivered intradermally. Avian and Bovine tuberculin will be referred to hereafter but it will be appreciated that the present invention will function as well if other tuberculins are used and the present invention is not limited to use with avian and bovine tuberculins.

The skin should be checked to ensure that a palpable nodule is present after each injection, the presence of a nodule indicates that the injection has been made intradermally.

The animal is then released from the crush / restraint and the next animal to be tested is brought into the crush. The whole of a herd are tested at the same time and it will be appreciated that for a large herd the process can take many hours to complete. Under the regulations having effect in the United Kingdom all the testing has to be performed at the same time and by the same individual.

Once each animal has been through the crush, the herd is left for a predetermined period (normally 72 hours plus or minus 4 hours after the injection of the tuberculin).

After the predetermined period, the second stage of the testing occurs. The animals are again taken into the crush one by one and the sites of the injections are inspected by eye and palpation of the skin, and the skin fold thickness measured again. The results of the inspection and comparison of fold thickness measurement will determine whether the animal being tested is regarded as infected with TB or not.

During the first stage of the testing two multiple use syringes are used, one for each tuberculin. The syringes used are such as those described in British patent GB622848 McLintock and are differentiated by one bearing a disc of a first colour, normally blue or red and the second bearing a disc of a second colour, normally red or blue. Each syringe further comprises a reservoir, a needle and an actuation means that will cause a predetermined volume of the contents of the reservoir to be pushed through the needle. The colour of the disc on the syringe is used to denote which tuberculin is in the syringe.

The syringes are held in holsters located on either hip of the person performing the test and it is standard practice that the person performing the test will always locate the bovine tuberculin on one side of his / her body and the avian tuberculin on the other side, for example bovine tuberculin is always on the right hip and avian tuberculin on the left hip. The person performing the test will use the hand on a given side of the body to inject the relevant skin patch with the tuberculin that is kept on that side of his / her body.

It is generally the case that the person performing the test will, before the start of the test and then at intervals during the testing, depending on how many animals are being tested, have to refill the reservoir of each syringe with the tuberculin appropriate for that syringe. The tuberculin is, prior to being taken into the reservoir of a syringe, stored in rubber topped vials so that the needle of the syringe can be pushed through the rubber top and used to draw the tuberculin into the reservoir.

Failure to correctly follow the testing procedure, for example by injecting the wrong tuberculin into the wrong skin patch (for whatever reason), not injecting each tuberculin intradermally, and / or not measuring skin thickness at the site of the intradermal injection may result in a false assessment as to whether a given animal has TB or not. The consequences of such a false assessment can be very serious including causing the destruction of a herd of healthy animals.

According to a first aspect of the present invention there is provided a syringe comprising a reservoir, a needle, a dispensing device suitable for dispensing a metered quantity of the contents of the reservoir via the needle, a means for actuating the dispensing device, and one or more of: a means for marking the position where the needle has entered the animal, a means for regulating the maximum possible depth of penetration of the needle into the animal, a means limiting the use of the syringe to one or other of the hands of the user, a means for monitoring the quantity of substance to be injected in the reservoir and/or a means to limit which substance to be injected may be loaded into the reservoir. The needle may be replaceable. The replacement of the needle may occur after a predetermined number of uses or when required, for example when not sufficiently sharp.

Where the syringe of the present invention is to be used for TB testing the substance to be injected is a tuberculin. Where the present invention is to be used for inoculations or dosing the substance to be injected is an inoculant or substance to be administered.

According to a second aspect of the present invention, there are provided a pair of syringes according to the first aspect of the present invention, the pair being so marked or configured that they are readily distinguishable by eye and not interchangeable. In one embodiment of this second aspect of the present invention, the whole or part of each syringe may be coloured so that each syringe may be readily differentiated from each other syringe by sight.

According to a third aspect of the present invention there is provided a syringe comprising a syringe frame with first and second syringes mounted on the syringe frame, each of the first and second syringes comprising a reservoir, a needle, a dispensing device suitable for dispensing a metered quantity of the contents of the reservoir via the needle, a means for actuating the dispensing device, and one or more of: a marker means for marking the position where the needle entered the skin, a first and second means for regulating the maximum possible depth of penetration of the needle into the skin, and / or a means for monitoring the quantity of substance to be injected present in the reservoir.

The means for actuating the first and second dispensing devices in the third aspect of the present invention may be so configured that the first and second dispensing devices are actuated simultaneously or in sequence.

The syringes of the first, second and third aspects of the present invention may be for use in connection with TB testing or for testing for other diseases, and / or treatment of animals or humans, the injection of medicines or supplements, and / or introduction of solid structures (for example a microchip or other identifying device) into the body of an animal or human. In embodiments of the present invention where the syringe of the present invention marks the position where the needle has entered the skin, the means of marking will preferably be a paint, ink or dye. The marking on the skin may be applied whist the needle is in the animal. The means of marking the skin may be via physical contact between a carrier of ink / paint / dye and the skin. The carrier could be a pad soaked in ink / paint / dye, a brush soaked in ink / paint / dye, or other structure carrying ink / paint / dye.

In one embodiment of the present invention, to ensure a good application of ink / paint / dye to the skin, the means of marking the skin is biased toward the skin and makes contact with the skin at around the same time as the tip of the needle. Pressure on the syringe to introduce the tip of the needle into the skin will cause the means of marking the skin to press against the skin so ensuring a suitable marking.

In alternative embodiments, the means of marking the skin may be via a spray of ink / paint / dye. Such a spray may be an aerosol but other forms of spray may be used.

In embodiments of the present invention where the means of marking the skin is a spray, a mask or stencil can be held next to the skin, optionally by the syringe, so as to ensure accurate marking of the position where the needle has entered the skin. The release of the spray can occur on contact of a pressure switch or similar device with the skin, or, in other embodiments can be actuated by the user, for example when the dispensing device is actuated.

It is most preferred that when a pair of syringes of the present invention are used in testing for TB or other disease each syringe is equipped with different coloured ink / paint / dye so that the position where the needle has entered the skin is marked with a colour which indicates which syringe was used at a particular point of injection. This has the considerable advantage that a look at the pair of skin patches or injection sites will confirm to a viewer that the correct syringe was used on each patch or site. Further, the coloured mark will allow the person checking for a palpable nodule to check in the correct location.

Alternatively, instead of or in addition to using different colours to differentiate between the syringes used at a position where the needle has entered the skin, a different shaped marking can be used, for example a star and a rectangle.

Where the syringe of the present invention is to be used in inoculating large numbers of animals, for example a flock of sheep, it is particularly advantageous if the syringe marks the animal when it is inoculated so that the person or persons performing the inoculations can see whether an animal has been inoculated or not.

In embodiments of the present invention where the syringe of the present invention is provided with a means for predetermining the maximum penetration of the needle into the skin the means is preferably physical and adjustable. In preferred embodiments of the present invention the means for setting the maximum depth of penetration is comprised of one or more posts, prongs, fingers or other structures (hereafter collectively "posts"), that have a longitudinal extent parallel to the needle. The ends of the posts are so located that when the end of the posts touch the skin the tip of the needle is at the predetermined depth. The or each post may be adjustable so that the position of the end of the or each post relative to the tip of the needle may be varied.

If it is desired that the needle is to enter the skin at an angle, that is not substantially perpendicular to the surface of the skin, then two or more posts can be provided either side of the needle, one being set differently from the other so that when the needle has entered the skin by desired distance and at the desired angle both posts contact the skin at substantially the same time.

In an alternative embodiment of the present invention a plate can be mounted on the syringe so that when the surface of the plate contacts the skin of the animal the needle has entered the skin to the correct depth. The plate can be orientated so that the face of the plate is substantially perpendicular to the axis of the needle or at an angle thereto.

In alternative embodiments of the present invention the means for determining the depth of penetration of the tip of the needle are retractable. In such embodiments, the means for determining the depth of penetration of the tip of the needle is biased into a first position such that the first part of the syringe that contacts the skin of the animal is the means for determining the depth of penetration of the tip of the needle. The user applies pressure to the syringe pushing it towards the skin and the needle advances into the skin whilst the end of the means for determining the depth of penetration of the tip of the needle retracts until a buffer or stop prevents further retraction. At this time the tip of the needle is at the correct depth in the skin. In these embodiments it is most preferred that the means for determining the depth of penetration of the tip of the needle is biased toward the first position by suitable springs.

Use of means for determining the depth of penetration of the tip of the needle when testing for TB is advantageous because it lessens the risk that the tuberculin is injected other than intradermally. A further advantage is that the means for determining the depth of penetration of the tip of the needle will also offer the needle of the syringe a degree of protection from damage from sideways (relative to the needle) impacts while the syringe is not in use.

In embodiments of the first and second aspects of the present invention where there is a means limiting the use of the syringe to one or other of the hands of the user, it is preferred that the syringe is so configured that the syringe has to be operated in a particular orientation relative to the users hand and it includes a physical barrier preventing use of the syringe with one of the users left or right hands. In one embodiment of the present invention the syringe is so configured that it is held via a pistol grip, one or more of the fingers holding that grip operates the syringe, and one side of the pistol grip is provided with a shield which prevents the grip being held from the side on which the shield is located. In such an embodiment, if the shield is located on the right hand side of the pistol grip then the grip can only be held with the left hand and if the shield is located on the left hand side of the grip, the pistol can only be held by the right hand.

In testing for TB or other diseases a pair of syringes according to these embodiments are used with one syringe being usable only with the left hand and the other syringe being usable only with the right hand. This will help to ensure that the syringes do not get mixed up by accident and the wrong tuberculin injected into the wrong skin patch.

In some embodiments of the present invention the syringe is provided with a means for monitoring the quantity of tuberculin or other substance to be injected that is present in the reservoir. The method of monitoring can be by measuring the quantity of tuberculin or other substance to be injected taken into the reservoir and the quantity exiting the reservoir via the needle. In some embodiments, the measuring means can be a flow meter. In such an embodiment, the syringe can be adapted so that the reservoir is refilled via the needle and there is a channel between the needle and the reservoir via the flow meter, and the flow meter records the volume of tuberculin flowing into the reservoir and, subsequently out of the reservoir and back to the needle. The flow meter can give a visual indication of the volume of the tuberculin or other substance to be injected in the reservoir either in a digital or analogue fashion. The flow meter additionally or alternatively may raise an alarm or cause the syringe to cease to operate when the syringe reservoir is close to empty.

In other embodiments, the syringe can be configured to count the number of doses of tuberculin or other substance that has been injected, and raise an alarm or cease to operate once a predetermined number of doses have been injected. The predetermined number can be set by the user when the tuberculin or other substance to be injected is introduced into the reservoir. This is advantageous because tuberculin or other substance to be injected can be supplied in different sized vials which contain different numbers of doses. The setting of the predetermined number of doses can be by direct reference to the volume of the tuberculin or other substance to be injected in the vial or on the basis of how many doses of tuberculin or other substance to be injected a vial contains.

In other embodiments the syringe of the present invention is provided with one or more means for measuring the weight of the content of the reservoir. Such means may be load cells located around the reservoir coupled to a processing unit that can, from the output of the load cells, calculate the weight of the reservoir and contents and thus the weight of the content of the reservoir. In one embodiment, the load cells are so arranged that they can calculate the weight of the reservoir irrespective of the orientation of the syringe. In another embodiment, the load cells are so arranged that they can calculate the weight of the reservoir when the syringe is in one predetermined orientation, for example when the syringe is in the holster on the hip of the user. The syringe can be adapted to raise an alarm or cease to operate when the weight of the reservoirs is at or below a predetermined value.

In some embodiments of the present invention the syringe of the present invention is provided with means to limit which tuberculin or other substance to be injected may be loaded into the reservoir. Such means to limit which tuberculin or other substance to be injected may be loaded into the reservoir are preferably comprised of a first part incorporated with the syringe of the present invention, and a second part which can be associated with the vial or vials in which the tuberculin or other substance is supplied and stored. The first and second parts of the means to limit which tuberculin or other substance to be injected may be loaded into the reservoir are preferably so dimensioned and configured that tuberculin or other substance to be injected can only be transferred into the reservoir from a vial if the first and second parts interact or engage with each other. It is most preferred, particularly in the context of TB testing, that the dimensions and configuration of the first and second parts for avian tuberculin are different to those for bovine tuberculin and the first part for avian tuberculin cannot interact with the second part for bovine tuberculin and visa-versa. In the context of or other substances to be injected it is likewise desired that substances do not erroneously get introduced into the wrong syringe. It is preferred that the second part of the means to limit which tuberculin or other substance to be injected may be loaded into the reservoir for each of the avian and bovine tuberculins or other substance to be injected are attached to the vials of the correct tuberculin or other substance to be injected before the testing commences, preferably before the vials of tuberculin or other substance to be injected are taken to the place where testing is to occur. Optionally, the vials can be manufactured so that he second parts are integral to the vial. This will prevent accidental attachment of the wrong second part to the vial. Alternatively, the vial(s) of tuberculin or other substance to be injected may be placed within a container which has an integral second part. This embodiment may further include insulation in the container so as to keep the vials of tuberculin or other substance to be injected at optimal temperature.

In an exemplary embodiment of the present invention one of the first or second parts may comprise a solid key of predetermined cross section, for example a rectangle or a circle, and the other of the first and second part a rigid sleeve where the internal shape and dimensions of the sleeve are such that the solid key may enter the sleeve. For a pair of syringes according to this embodiment of the present invention one syringe will have a first solid key of a first cross section, for example a cross section of a circle, and the second a cross section of a rectangle. The circle and rectangle will be so dimensioned that that the circle and rectangle keys cannot fit into the rectangle and circle sleeves respectively. For example, the circle cross section solid key might be of 6mm diameter and the rectangular cross section solid key might be 8mm by 4mm.

The first parts of the means to limit which tuberculin or other substance may be loaded into the reservoir are in one embodiment of the present invention so configured that they surround the base of the needle and the second part so configured that the needle cannot be pushed through the rubber top of the vial of tuberculin or other substance to be injected unless the first part has entered the sleeve of the second part.

Other possible configurations of first and second parts of the means to limit which tuberculin may be loaded into the reservoir may be envisaged and are included in the scope of the present invention.

In other embodiments of the present invention the reservoir associated with each syringe of the present invention is comprised of a vial of tuberculin or other substance to be injected such as that obtained from the manufacturers of the tuberculin or other substance to be injected. In such embodiments of the present invention the syringe may include a receptacle into which a vial of tuberculin or other substance to be injected may be placed, and means for creating a liquid communication between the contents of the vial and the needle of the syringe. In such embodiments of the present invention it is most preferred that the receptacles for the vials of each tuberculin or other substance to be injected are differently shaped, dimensioned or otherwise differentiated so that if the vials for the different tuberculins or other substances to be injected are differently shaped, dimensioned or otherwise differentiated, each receptacle will only accept a vial for a predetermined tuberculin or other substance to be injected. This will avoid the wrong tuberculin or other substance to be injected being placed in the wrong syringe.

A further advantage of this embodiment of the present invention is that because the tuberculin or other substance to be injected is not being transferred from the vial in which it is supplied to a reservoir there is a reduced risk of contamination of the tuberculin or other substance to be injected and / or that the wrong tuberculin or other substance to be injected will be placed in the wrong reservoir.

In some embodiments of the present invention the tuberculin or other substance to be injected is supplied from the manufacturer in a tube or a straw and the or each syringe of the present invention is adapted to receive that tube or straw. The tube or straw may, in some embodiments, be constructed of a material which is flexible and allows a quantity of tuberculin, inoculant or other substance to be squeezed out of the straw on the actuation of the dispensing means.

In other embodiments of the present invention the straw or tube may be constructed from a rigid or semi-rigid material and is so configured that a plunger may be driven along at least part of the length of the tube or straw to expel a quantity of the tuberculin, inoculant or other substance when the dispensing means is actuated. Preferably the plunger is integral with the tube or straw when supplied by a manufacturer.

In these embodiments, if it is desired to supply the tuberculin, inoculant or other substance from the manufacture in a number of different volumes the straw or tube may of different lengths for different volumes. Alternatively, the straws or tubes may all be of the same length and the size of the plunger is different for each volume. This has the advantage that the part of the syringe that drives the plunger forward on actuation of the dispensing means can always be set to start in the same position once a new straw or tube is loaded into a syringe according to the present invention.

An advantage of these embodiments of the present invention is that once the plunger has moved along the straw or tube and expelled all of the tuberculin, inoculant or other substance from the straw or tube the plunger will impact the end of the straw or tube remote from the end that the plunger started and not be able to advance any further. If the means for actuating the dispensing means is mechanical, powered by the user, and mechanically linked to the means for driving the plunger forward, the user will be able to feel that the plunger cannot advance further and thus know immediately that the content of the straw or tube has been fully emptied from the straw or tube. In other embodiments, the impact of the plunger on the end of the straw or tube will be reported to a user of the syringe by means other than the feel of the dispensing means actuation means.

In these embodiments the straws or tubes are preferably of different sizes, shapes and or appearances for different tuberculins, inoculants or other substances.

In an alternative embodiment of the present invention, the reservoir for each syringe may be comprised of a fluid linkage which is adapted to engage with a vial of tuberculin or other substance to be injected. In such embodiments, the fluid linkage may be at least one, and preferably two conduits, tubes or pipes that are provided with means to engage with a vial of tuberculin or other substance to be injected. The means for engagement with the vial may be so configured that the means can only engage with a vial of a particular tuberculin or other substance to be injected. In one embodiment the means for engagement for a particular syringe can be a male / female coupling where the male element of the coupling is located on the or each fluid linkage and the female element on a vial of a particular, for example bovine, tuberculin or other substance to be injected. The other syringe of the second or third aspects of the present invention would be so configured that the female element of the coupling is located on the or each fluid linkage and the male element on a vial of a particular, for example avian, tuberculin or other substance to be injected.

In one embodiment of this embodiment of the present invention a first conduit is adapted to transfer tuberculin or other substance to be injected from inside the vial to the needle for injection and the second conduit is adapted to introduce gas (which may be the atmosphere or a sterile gas such as carbon dioxide, nitrogen other gas) into the vial to replace the volume of tuberculin or other substance to be injected withdrawn from the vial or to push the tuberculin or other substance to be injected out of the vial. In alternative embodiments, the means for introducing gas in to the vial may be different. In still further alternative embodiments, the vial may be so constructed that it reduces in volume when the contents of the vial is withdrawn from the vial.

It will be readily appreciated that use of a pair of syringes according to the first, second or third aspects of the present invention will lower the chances of errors in the course of TB testing and increase the ability of the person performing the test and any onlookers identifying any errors that are made at the time they are made so that they can be recorded and rectified. This will lead to a decreased rate of false results and lessen the distress and economic impact of erroneously identifying animals as having TB when they do not.

The present invention will be further described and explained by way of example. Such examples are not limiting as to the scope of the present invention. The examples include reference to the accompanying figures in which:
Figure 1 shows a schematic view of an embodiment of a syringe according to the present invention;
Figure 2 shows an enlarged view of the shield, trigger and grip portion of the syringe of Figure 1;
Figure 3 shows an enlarged view of the end of the syringe of Figure 1 remote from the grip;
Figure 4 shows an embodiment of a needle adapted to be fitted to the end of the syringe of
Figure 3;

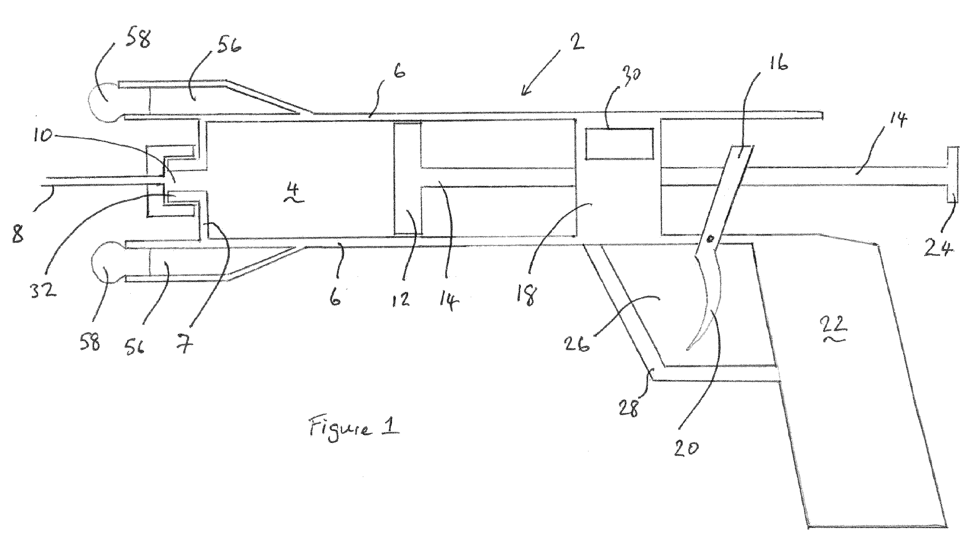

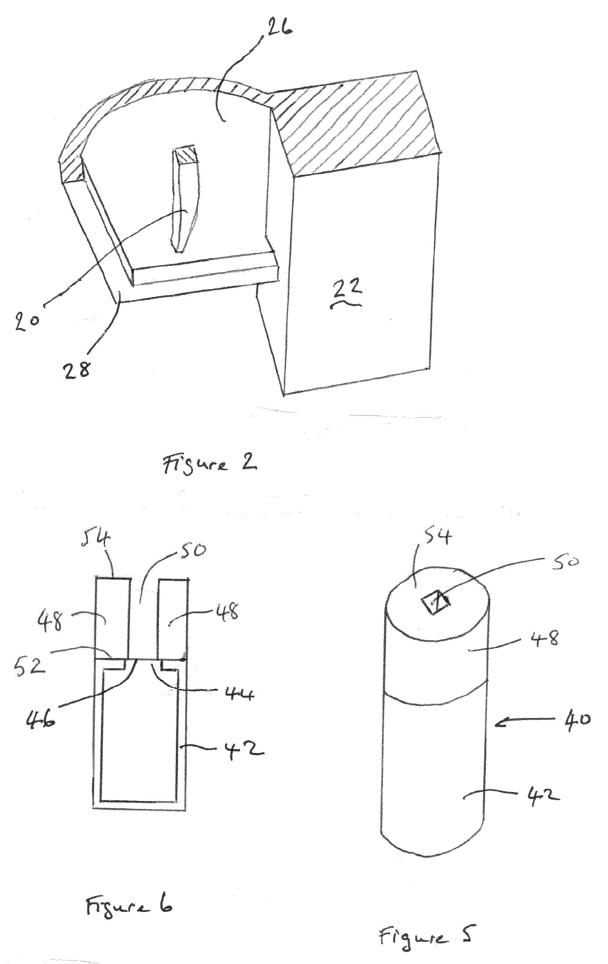

Figure 5 shows a perspective view of a vial for use in association with the syringe of Figure 1;
Figure 6 shows a sectional view of the vial of Figure 5; and
Figure 7 shows the markings made on the skin of an animal or human by the syringe of Figure 1 when it is used.

With reference to Figure 1, a syringe (2) comprises a reservoir (4) defined by a cylindrical wall (6), an end wall (7), and a plunger (12). In fluid communication with the reservoir (4) via a channel (10) through the end wall (7) is an introduction means in the form of a hypodermic needle (8). Channel (10) passes through the end wall (7).

Plunger (12) is caused to decrease the volume of the reservoir (4) when a dispensing means is actuated. The dispensing means is comprised of the plunger (12), a plunger shaft (14), a trigger lever (16) that is loosely engaged with the plunger shaft (14), and a support (18). The dispenser actuation means is a trigger (20).

The end of the plunger shaft (14) adjacent the plunger (12) is connected to the plunger (12) by a known method so that the plunger shaft (14) can either push the plunger (12) to decrease the volume of the reservoir (4) or pull the plunger (12) to increase the volume of the reservoir (4). That end of the plunger shaft (14) is held relative to the plunger (12) to prevent movement in a direction not substantially parallel to the main axis of the plunger shaft (14) - lateral movement. Support (18) defines a linear bore passing through the support (18) along which the plunger shaft passes. The bore has a main axis substantially coincident with the main axis of the plunger shaft and is dimensioned to allow movement of the plunger shaft in the direction of its longitudinal axis but to restrain the plunger shaft (14) from lateral movement.

The trigger lever (16) is rigidly connected with the trigger (20) and the combined trigger lever and trigger assembly is pivotally connected to the wall (6), or a mounting block (not shown) which is connected to the wall (6). The pivot is located between the trigger (20) and the position of engagement of the trigger lever (16) and the plunger shaft (14). Also connected to wall (6) or integral therewith is a grip or handle (22) which in this embodiment is configured as a pistol grip. The trigger (20) is spaced from the grip (22) so that a user holding the grip (22) can pull the trigger (20) with one of the fingers of the hand holding the grip (22).

The loose engagement of the plunger shaft (14) and the trigger lever (16) is via a ratchet mechanism (not illustrated). The ratchet mechanism includes a linear rack (not illustrated) attached to and extending along at least a portion of the length of the plunger shaft (14) with the rack being so positioned and orientated that a pawl (not illustrated) which is fixed to the trigger lever (16) may, when a user pulls the trigger (20) towards the handle (22), engage with teeth of the rack and push the plunger shaft (14) and the plunger (12) towards the end wall (7) causing the plunger (12) to decrease the volume of the reservoir (4). Means (not shown) are also provide which allow a user to disengage the pawl from the rack so that the user can pull, using a grip (24), the plunger shaft (14) and plunger (12) away from the end wall (7) so as to increase the volume of the reservoir (4). This increase in volume can be used to suck a substance to be injected into the reservoir via the needle (8) and the channel (10).

Further with regard to the trigger (20) and the grip (22), and with reference to Figure 2, there is further provided a shield (26) which extends between a trigger guard (28), the grip (22), and the wall (6). The shield (26) is solid and has the effect that the trigger (20) can only be operated with a finger of the users left or right hand but not both. In Figures 1 and 2, the hand that will be able to hold the grip and pull the trigger is the left hand. In alternative embodiments of the present invention the shield will be located on the other side of the trigger and in that instance the grip (22) will be held with the right hand and the trigger (20) pulled with a finger of that hand.

Also provided in association with the trigger (20) and trigger lever (16) are means (not shown) for limiting the distance of travel of the trigger (20) when pulled by a user, this has the effect that the distance that the plunger shaft is driven by the pulling of the trigger (20) is likewise limited. This allows a known reduction in the volume of the reservoir each time the trigger (20) is pulled and the trigger travels the maximum distance it may travel. As a result, a known dose of substance to be injected is dispensed each time the trigger is pulled. The means for limiting the distance of travel of the trigger (20) may be adjustable so that the size of the dose dispensed each time the trigger (20) is pulled may be adjusted.

The support (18) may further include a means for monitoring the quantity of the substance to be injected that is in the reservoir (4). In the embodiment of the present invention illustrated in Figure 1, the means for monitoring includes a gear wheel that engages with the teeth of the linear rack (not illustrated) that is attached to the plunger shaft (14), rotation of said gear wheel causing a mechanical or electronic counting mechanism to increase or decrease the value held in that counting mechanism depending upon the direction of rotation of the gear wheel. The value held in the counting mechanism can be used to derive, again electronically or mechanically, the volume of substance in the reservoir, the number of injections left in the reservoir, or other data that may be of use to a user. That derivative value is displayed in the display (30). Display (30) may be electronic or mechanical.

The counting mechanism may be calibrated so that when the plunger (12) abuts the end wall (7) the value in the counting mechanism is zero. Pulling the plunger shaft (14) / plunger (12) away from the end wall (7) will cause the value in the mechanism to increase. Movement of the plunger shaft (14) / plunger (12) towards the end wall (7) towards the end wall (7) causes the value to decrease.

The syringe (2) of the embodiment of the present invention illustrated in Figure 1 also includes means for marking the skin of the animal when a dose of the substance to be administered is administered. These means comprise a first and second colourant reservoir (56) suitable for containing a colourant and a first and second colourant application means (58) of sponge or like material. The colourant application means is in fluid communication with the content of the colourant reservoir.

The colourant application means (58) are so located relative to the needle (8) that when the syringe (2) is being used to administer to an animal or human a dose of a substance to be administered and the needle has entered the skin of the animal or human to the correct depth the colourant application means (58) contacts the skin of the animal or human and makes a mark thereon. With the syringe (2) illustrated in Figure 1 the marks made on the skin will be as shown in Figure 7, where the marks (60) are made by the colourant application means (58), and as a result it is known that the needle (8) entered the skin at position (62). The position of the colourant application means (58) may be adjustable in some embodiments of the present invention. The colourant applied may be paint, ink, dye or another appropriate substance or a mixture of two or more of these substances.

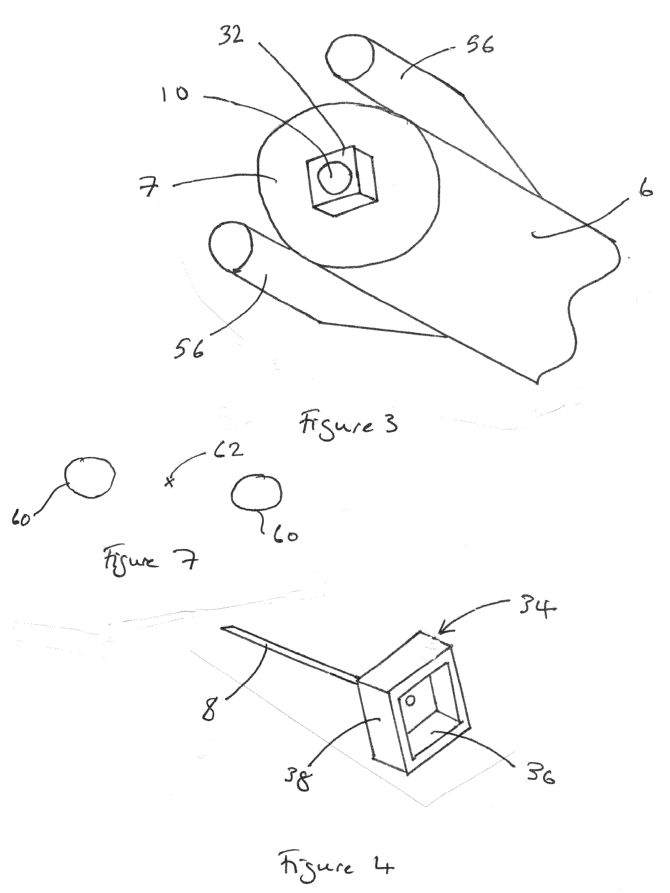

With reference to Figures 3 and 4, on the face of the end wall (7) remote from the plunger (12) is located a boss (32). The channel (10) passes through the end wall (7) and the boss (32). The boss (10) has a shape that is adapted to be a male push fit with the inside face (36) of a female connector (34) at the base of the hypodermic needle (8) and the female connector is held in place by friction. The outside face (38) of the female connector (34) has, in the illustrated embodiment, the same shape as the inner face (36). In alternative embodiments, the shapes of the inner face (36) and outer face (38) can differ. In either event, according to the present invention the shape of the boss (32) and inner face (36) is a shape that is not used in the standardized Luer taper connectors commonly used for hypodermic needles.

In further alternative embodiments of the present invention the male boss (32) and the female connector (34) are so configured to include a positive lock or engagement mechanism between them.

With reference to Figures 5 and 6, a vial (40) for the storage and supply of a substance to be administered is comprised of a storage container (42) with a mouth (44) which is closed by a seal (46) of rubber or other known and appropriate material that may be pierced by the needle (8). Attached to the portion of the storage container (42) that defines the mouth (44) is a barrel (48). The barrel (48) defines a channel (50). The channel (50) is substantially linear and extends from a mouth in the surface (52) of the barrel (48) that overlies the seal (46) to the opposite face (54) of the barrel (48) and mouths through the face (54). The channel (50) has a cross-section in the plane perpendicular to the longitudinal axis of the channel (50) that is the same as the shape of the outside face (38) of the female connector (34) in substantially the same plane when the axis of the needle (8) is substantially parallel to the longitudinal axis of the channel (50). The dimensions of the barrel (48) and the needle (8) are such that when the female connector (34) is not at least partially within the channel (50) the needle (8) is not of sufficient length to reach the seal (46). When the female connector (34) is inserted into the channel (50) to at least a certain degree, for example half way in, the needle (8) will pierce the seal (46). This will allow the content of the storage container (42) to be drawn into the reservoir (4).

When both a first and second syringe (2) of the present invention are to be used together, for example in testing for bovine tuberculosis, it is preferred that the shape or dimensions of the male boss (32), the inner and outer faces (36, 38) of the female connector (34) of the first and second syringes, and the cross-section of the channel (50) of a vial associated with the first and second syringes differ. Most preferably, the shapes are such that the outer face (38) of the female connector (34) of the first syringe will not fit into the channel (50) of the vial associated with the second syringe, and the outer face (38) of the female connector (34) of the second syringe will not fit into the channel (50) of the vial associated with the first syringe. This has the effect that a user is prevented from putting the wrong tuberculin in the wrong syringe.

Statements of invention:
1 A syringe for injecting into or through the skin of an animal or human comprising a reservoir adapted to contain a substance to be administered to the animal or human, an introduction means for introducing the substance to be administered into the animal or human, a dispensing means suitable for dispensing a dose of the content of the reservoir from the reservoir into the animal or human via the introduction means, and a means for actuating the dispensing means, characterised in that the syringe further comprises one or more of a means limiting the use of the syringe to one or other of the hands of the user,
   a means for monitoring the quantity of substance in the reservoir,
   a means to limit which substance may be introduced into the reservoir, or
   the reservoir is reversibly removable from the syringe,
   or the syringe further comprises a means for marking the position where the introduction means has entered the skin and one or more of
   a means limiting the use of the syringe to one or other of the hands of the user,
   a means for monitoring the quantity of substance in the reservoir,
   a means to limit which substance may be introduced into the reservoir, or
   the reservoir is reversibly removable from the syringe
2 A syringe according to statement 1 in which the introduction means is a needle.
3 A syringe according to statement 1 or 2 in which the syringe is provided with a means for marking the position where the introduction means causes the substance to be administered to enter into the animal or human, the position being marked with a colourant.
4 A syringe according to statement 3 in which the marking means comprises a reservoir of colourant and an applicator, and when the introduction means causes the substance to be administered to enter into the animal or human the applicator makes contact with the skin or hair covering the skin, and applies colourant thereto.
5 A syringe according to statement 4 in which the applicator is configured to make a specifically shaped mark on the skin.
6 A syringe according to statement 3 in which the skin marking means comprises a reservoir of colourant and an applicator and the applicator is a means for spraying the colourant onto the skin or hair covering the skin when the introduction means is introducing the substance to be administered into the animal or human.
7 A syringe according to statement 6 in which the syringe means further comprises a means for actuating the applicator when the introduction means causes the substance to be administered to enter into the animal or human.
8 A syringe according to statement 7 in which the applicator is actuated by the means for actuating the dispensing means.
9 A syringe according to any of statements 6 to 8 in which the skin marking means further comprises a stencil or mask suitable for shaping the mark made by the skin marking means.
10 A syringe according to any of statements 3 to 9 in which the colourant is comprised of at least one of an ink, a dye, or a paint.
11 A syringe according to any of statements 1 to 10 in which the syringe includes a means for monitoring the quantity of substance to be injected in the reservoir.
12 A syringe according to statement 11 in which the means for monitoring the quantity of substance to be injected in the reservoir is comprised of a means for measuring the quantity of substance that is introduced into the reservoir, a means for measuring the quantity of substance that is removed from the reservoir, and a means for subtracting the quantity of substance that is removed from the quantity of substance that is introduced.
13 A syringe according to statement 12 in which the quantity is measured in terms of one of volume, mass or weight.
14 A syringe according to tatement 12 or 13 in which the syringe further comprises a display means and the output of the means for subtracting the quantity of substance that is removed from the quantity of substance that is introduced is displayed on the display means.
15 A syringe according to any of statements 12 to 14 in which the syringe further comprises a means that takes the output of the means for subtracting the quantity of substance that is removed from the quantity of substance that is introduced, compares that output to one or more predetermined criteria, and makes the user aware of the result of the comparison if one or more of the predetermined criteria are met.
16 A syringe according to statement 11 in which the means for monitoring the quantity of substance to be injected in the reservoir is comprised of a means for a user to input the number of doses of substance introduced into the reservoir, a means for counting the number of doses of substance dispensed via the introduction means, and a means for calculating the number of doses left in the reservoir.
17 A syringe according to statement 16 in which the syringe further comprises a display means and the number of doses left in the reservoir is displayed on the display means.
18 A syringe according to statement 16 or 17 in which the syringe further comprises a means for comparing the number of doses left in the reservoir with one or more predetermined criteria, and makes the user aware of the result of the comparison if one or more of the predetermined criteria are met.
19 A syringe according to any of statements 11 to 18 in which the syringe further comprises a means for preventing operation of the dispensing means when the amount of content of the reservoir is lower than a predetermined level.
20 A syringe according to statement 11 in which the means for monitoring the quantity of substance to be injected in the reservoir is comprised of a means for counting the number of doses of substance dispensed, and a means for displaying the number of doses of substance dispensed.
21 A syringe according to any of statements 1 to 20 which includes a means to limit which substance may be introduced into the reservoir.
22 A syringe according to statement 21 in which the means to limit which substance may be introduced into the reservoir comprises a first part incorporated with the syringe, the first part being suitable for engagement with a second part which is incorporated in or associated with a vial for a predetermined substance, the first and second parts being so dimensioned and configured that the substance can only be transferred from the vial to the reservoir when the first and second parts are engaged with each other.
23 A syringe according to statement 22 in which the first part is a solid shape and the second part is a sleeve configured and dimensioned so that the solid shape of the first part is a sliding or loose fit within the sleeve of the second part.
24 A syringe according to any of statements 1 to 20 in which the syringe further comprises a means for reversibly connecting the reservoir with the dispensing means and or introduction means and in which the reservoir is reversibly removable from the syringe.
25 A syringe according to statement 24 in which the reservoir is comprised of a container which may be engaged with the syringe when the container is at least partially filled with substance to be injected and disengaged from the syringe when a user wishes to do so.
26 A syringe according to statement 24 or 25 in which the means for reversibly connecting the reservoir with the dispensing means and or introduction means includes a means to limit which reservoir may be connected with the syringe.
27 A syringe according to statement 26 in which the means to limit which reservoir may be connected with the syringe comprises a first part incorporated with the syringe, the first part being suitable for engagement with a second part which is incorporated in or associated with the reservoir, the first and second parts being so dimensioned and configured that the reservoir can only connect with the syringe when the first and second parts are engaged with each other.
28 A syringe according to statement 27 in which the first part is a solid shape or sleeve and the second part is a sleeve or solid shape configured and dimensioned so that the solid shape is a sliding or loose fit within the sleeve.
29 A syringe according to any of statements 21 to 28 in which the reservoir is a vial in which the substance to be injected was placed when it is was manufactured or packaged following manufacture.
30 A syringe according to any of statements 1 to 29 in which the syringe further comprises a means for visually differentiating the syringe from other syringes.
31 A syringe according to statement 30 in which the visual differentiation means is one or more of a colour, a shape attached to the syringe, letters or numbers applied to the syringe.
32 A pair of syringes according to statement 30 or 31 in which the visual differentiation means is different for each syringe.
33 A syringe according to any of statements 1 to 32 in which the syringe is so configured that it can only be operated with the left hand of a user.
34 A syringe according to any of statements 1 to 32 in which the syringe is so configured that it can only be operated with the right hand of a user.
35 A vial for containing a substance for injection using a syringe according to any of statements 22, 23, 27, 28 or 29 in which the vial is comprised of a vial body including a mouth through which the substance may be accessed, characterised in that the mouth is dimensioned and configured to form the second part.
36 A temporary housing for one or more vials for containing a substance for injection using a syringe according to statements 22, 23, 27, 28 or 29 in which each vial has a body and a mouth, the housing is comprised of a housing body defining a store space in which one or more vials may be stored in a predetermined configuration, and the housing body is provided with at least one aperture through the housing body, the or each aperture allowing access to the mouth of a vial located in the store space, characterised in that the aperture is dimensioned and configured to form the second part.
37 A needle for use in association with a syringe according to statement 22 or 23 in which the needle is comprised of a tubular shaft and a base, in which the base is adapted to releasably connect to at least one element of the syringe characterised in that the outer dimensions of the base are dimensioned and configured to form the first part.
38 Use of a syringe according to any of statements1 to 34 in injecting an animal or human with a substance.
39 Use of a pair of syringes according to any of statements1 to 34 in the performance of testing for tuberculosis in animals.

## Claims

1. A syringe for injecting into or through the skin of an animal or human comprising a reservoir adapted to contain a substance to be administered to the animal or human, an introduction means for introducing the substance to be administered into the animal or human, a dispensing means suitable for dispensing a dose of the content of the reservoir from the reservoir into the animal or human via the introduction means, and a means for actuating the dispensing means, **characterised in that** the syringe further comprises one or more of a means limiting the use of the syringe to one or other of the hands of the user,
a means for monitoring the quantity of substance in the reservoir,
a means to limit which substance may be introduced into the reservoir, or
the reservoir is reversibly removable from the syringe,
or the syringe further comprises a means for marking the position where the introduction means has entered the skin and one or more of
a means limiting the use of the syringe to one or other of the hands of the user,
a means for monitoring the quantity of substance in the reservoir,
a means to limit which substance may be introduced into the reservoir, or
the reservoir is reversibly removable from the syringe

2. A syringe according to claim 1 in which the introduction means is a needle.

3. A syringe according to claim 1 or 2 in which the syringe is provided with a means for marking the position where the introduction means causes the substance to be administered to enter into the animal or human, the position being marked with a colourant and the marking means comprises a reservoir of colourant and an applicator.

4. A syringe according to claim 3 in which the applicator is configured to make a specifically shaped mark on the skin or the marking means further comprises a stencil or mask suitable for shaping the mark made by the applicator.

5. A syringe according to any of claims 1 to 4 in which the syringe includes a means for monitoring the quantity of substance to be injected in the reservoir or the quantity of substance to be injected that leaves the reservoir, and a means for displaying that quantity or a quantity of that quantity or a derivation of that quantity to a user.

6. A syringe according to claim 5 in which the syringe further comprises a means for comparing the quantity of substance that is remaining in or has left the reservoir and making the user aware of the result of the comparison if one or more predetermined criteria are met by that comparison.

7. A syringe according to any of claims 1 to 6 which includes a means to limit which substance may be introduced into the reservoir.

8. A syringe according to claim 7 in which the means to limit which substance may be introduced into the reservoir comprises a first part incorporated with the syringe, the first part being suitable for engagement with a second part which is incorporated in or associated with a vial for a predetermined substance, the first and second parts being so dimensioned and configured that the substance can only be transferred from the vial to the reservoir when the first and second parts are engaged with each other.

9. A syringe according to any of claims 1 to 8 in which the syringe further comprises a means for reversibly connecting the reservoir with the dispensing means and introduction means so that the reservoir is reversibly removable from the syringe, and
the means for reversibly connecting the reservoir with the dispensing means and introduction means includes a means to limit which reservoir may be connected with the syringe.

10. A syringe according to any of claims 1 to 9 in which the syringe is so configured that it can only be operated with one of the users hands in normal use.

11. A vial for containing a substance for injection using a syringe according to any of claims 7, 8, and 9 in which the vial is comprised of a vial body including a mouth through which the substance may be accessed, **characterised in that** the mouth is dimensioned and configured to form the second part.

12. A temporary housing for one or more vials for containing a substance for injection using a syringe according to claim 7 or 8 in which each vial has a body and a mouth, the housing is comprised of a housing body defining a store space in which one or more vials may be stored in a predetermined configuration, and the housing body is provided with at least one aperture through the housing body, the or each aperture allowing access to the mouth of a vial located in the store space, **characterised in that** the aperture is dimensioned and configured to form the second part.

13. A needle for use in association with a syringe according to claim 8 in which the needle is comprised of a tubular shaft and a base, in which the base is adapted to releasably connect to at least one element of the syringe **characterised in that** the outer dimensions of the base are dimensioned and configured to form the first part.

14. Use of a syringe according to any of claims 1 to 13 in injecting an animal or human with a substance.

15. Use of a pair of syringes according to any of claims 1 to 13 in the performance of testing for tuberculosis in animals.
